# EUROPEAN PATENT APPLICATION

(11) **EP 4 389 126 A1**
(43) Date of publication of application: **26.06.2024**
(21) Application number: 21954316.2
(22) Date of filing: 20.08.2021
(51) Int. Cl.: A61K 31/445, A61P 25/18, A23L 33/10, G01N 33/68, C12Q 1/6883

(54) **PHARMACEUTICAL COMPOSITION FOR PREVENTING OR TREATING ATTENTION DEFICIT/HYPERACTIVITY DISORDER**

(71) Applicant: Dankook University Cheonan Campus Industry Academic Cooperation Foundation, Chungcheongnam-do 31116 (KR); Institute for Basic Science, Daejeon 34126 (KR)
(72) Inventor: YOON, Bo-Eun, Yongin-si Gyeonggi-do 16917 (KR); LEE, C. Justin, Daejeon 34125 (KR); KIM, Yoo Sung, Cheonan-si Chungcheongnam-do 31119 (KR); SA, Moonsun, Daejeon 34199 (KR)
(74) Representative: Mewburn Ellis LLP
(86) International application number: PCT/KR2021/011136
(87) International publication number: WO 2023/022269

(57) **Abstract**

The present invention relates to a pharmaceutical composition for preventing or treating attention deficit/hyperactivity disorder (ADHD), comprising SNAP5114 as an active ingredient. A *Git1* gene-deficient hetero (+/-) mouse, confirmed to have increased amount of GABA in the brain striatum that controls hyperactivity, can be used as an animal model of ADHD, and by confirming that hyperactivity is ameliorated by administering SNAP5114 to the *Git1* gene-deficient hetero (+/-) mouse, SNAP5114 is provided as a therapeutic agent for ADHD.

## Description

### Technical Field

The present disclosure relates to a pharmaceutical composition for preventing or treating attention deficit/hyperactivity disorder (ADHD).

### Background Art

Attention deficit/hyperactivity disorder (ADHD) is one of the most common mental disorders that first appears in childhood, and it may also occur in adulthood and throughout adulthood. It has been reported that approximately 4.1% of children between the ages of 9 and 17 suffer from ADHD. Infants with ADHD are unable to concentrate on any task and sit quietly, but act impulsively with an inability to complete a task. If left untreated, infants are more likely to be injured, and the disorder has long-term adverse effects on an ability to make friends and a capability in school and/or study of the affected children. Over time, the child with ADHD is more likely to develop depression, poor self-esteem, and other emotional problems.

In most cases, infants and adults with ADHD are treated with psychostimulants such as amphetamines, methylphenidate, and pemoline. Further, antidepressants such as desipramine, which selectively blocks the reuptake of norepinephrine, are also effective in some cases. In addition, new drugs such as atomoxetine that blocks the reuptake of norepinephrine and serotonin may also be effective in treating the disorders. Psychostimulants and monoamine reuptake inhibitors control activity levels and attention, but they are not effective in treating the cognitive deficits associated with or accompanying ADHD.

Causes of ADHD that have been cited include genetic factors, biochemical factors such as lead levels and side reactions to food additives in instant foods, views that there are defects in choosing the right stimuli to the brain, environmental factors such as the relationship between parents and children and the social status of parents, and maternal smoking and alcohol abuse during pregnancy, but the causes are still on the debate. However, neurobiological factors are considered more important than psychosocial factors, and thus research on drug treatment and biological factors for this disease is actively conducted. In particular, it has been suggested that it may be a non-uniform group of diseases caused by abnormalities in the interrelation of various brain regions responsible for higher cognitive functions, rather than abnormalities in the development of a single nervous system due to biological factors. Structural and functional brain imaging studies on children with ADHD up to date have revealed that the pathophysiology of ADHD is generally associated with dysfunction in the fronto-striatal tract, and the drug effect of methylphenidate (MPH) is associated with functional changes in the dopaminergic system in this region. In addition to dopaminergic neurons, symptoms of ADHD are related to the regulation of noradrenaline neurons, which control neural circuits in the frontal lobe. Studies have also reported that ADHD behavior is caused by an imbalance in the regulation of noradrenaline and dopaminergic neurons. Broadly speaking, it is understood that the behavior is attributable to a reduced influence of catecholamines, which dominate glutamatergic neurons and g-aminobutyric acid (GABA) neurons, on the posterior synapse region.

### [Prior Art Document]

### [Patent Document]

Korean Patent Application Publication No. 10-2005-0085538 (published on August 29, 2005)

### Disclosure of the Invention

### Technical Goals

An object of the present disclosure is to provide a pharmaceutical composition for preventing or treating attention deficit/hyperactivity disorder (ADHD), including a component that exhibits an effect of ameliorating hyperactivity.

In addition, another object of the present disclosure is to provide a biomarker composition for diagnosing attention deficit/hyperactivity disorder (ADHD).

### Technical Solutions

The present disclosure provides a pharmaceutical composition for preventing or treating attention deficit/hyperactivity disorder (ADHD), including SNAP5114 or a pharmaceutically acceptable salt thereof as an active ingredient.

In addition, the present disclosure provides a preventive or health functional food composition for attention deficit/hyperactivity disorder (ADHD), including SNAP5114 and a foodologically acceptable food supplemental additive.

In addition, the present disclosure provides a biomarker composition for diagnosing attention deficit/hyperactivity disorder (ADHD), including GABA transporter-3 (GAT-3) or a gene encoding the same as an active ingredient.

In addition, the present disclosure provides a kit for diagnosing attention deficit/hyperactivity disorder (ADHD), including, as an active ingredient, a preparation capable of detecting an expression level of a GABA transporter-3 (GAT-3) protein or an expression level of a gene encoding the same.

In addition, the present disclosure provides a method of providing information for diagnosis of attention deficit/hyperactivity disorder (ADHD), including measuring an expression level of a GABA transporter-3 (GAT-3) protein or an expression level of a gene encoding the same in a biological sample isolated from a subject suspected of having attention deficit/hyperactivity disorder (ADHD); and comparing the expression level with that of a biological sample isolated from a normal person.

### Advantageous Effects

According to the present disclosure, *Git1* gene-deficient hetero (+/-) mice may be used as an animal model for attention deficit/hyperactivity disorder (ADHD) by identifying an increase in an amount of GABA in the striatum of the brain that controls hyperactivity, SNAP5114 may be provided as a therapeutic agent for attention deficit/hyperactivity disorder (ADHD) by identifying that hyperactivity is ameliorated upon administration of SNAP5114 into the *Git1* gene-deficient hetero (+/-) mice, and GABA transporter-3 (GAT-3) may be provided as a biomarker capable of diagnosing attention deficit/hyperactivity disorder (ADHD).

### Brief Description of Drawings

FIG. 1 shows results of evaluating changes in an amount of GABA in an animal model with attention deficit/hyperactivity disorder (ADHD) by fluorescent immunohistochemistry (fIHC).
FIG. 2 shows results of evaluating changes in an amount of GABA in an animal model with attention deficit/hyperactivity disorder (ADHD) by electrophysiological measurement (patch clamp recording).
FIG. 3 shows results of evaluating changes in hyperactivity upon administration of SNAP5114 in an animal model with attention deficit/hyperactivity disorder (ADHD). Best Mode for Carrying Out the Invention

The terms used herein are selected from general terms that are currently, widely used as much as possible in consideration of functions in the present disclosure, but they may vary depending on the intention or precedent of a person skilled in the art and the emergence of new technology. In addition, in certain cases, there are terms arbitrarily selected by the applicant, in which case their meanings will be described in detail in the corresponding description of the disclosure. Therefore, the terms used herein should be defined based on the meaning of the term and the overall content of the present disclosure, rather than simply the name of the term.

Unless otherwise defined, all terms used herein, including technical or scientific terms, have the same meanings as are generally understood by those skilled in the art to which the present disclosure pertains. Terms such as those defined in commonly used dictionaries should be construed as having meanings consistent with their meanings in the context of the relevant descriptions and should not be construed in an idealistic or excessively formal sense, unless clearly defined in the application.

A numerical range includes values defined in the above range. All maximum numerical limits given throughout the specification include all lower numerical limits, as clearly stated in the lower numerical limits. All minimum numerical limits given throughout the specification include all higher numerical limits, as clearly stated in the higher numerical limits. Any numerical limits given throughout the specification will include all better numerical ranges within a wider numerical range, as the narrower numerical limits are clearly stated.

### Hereinafter, the present disclosure will be described in detail.

The present disclosure provides a pharmaceutical composition for preventing or treating attention deficit/hyperactivity disorder (ADHD), including SNAP5114 or a pharmaceutically acceptable salt thereof as an active ingredient.

The SNAP5114 is a compound represented by the following Chemical Formula 1, specifically (S)-1-(2-(tris(4-methoxyphenyl)methoxy)ethyl)piperidine-3-carboxylic acid.

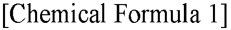

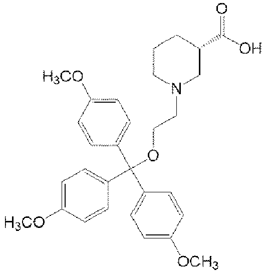

The SNAP5114 has an effect of ameliorating hyperactivity in an animal model with attention deficit/hyperactivity disorder (ADHD).

The pharmaceutically acceptable salts refer to acid additive salts formed by pharmaceutically acceptable free acids, and the pharmaceutically acceptable salts refer to salts commonly used in the pharmaceutical industry, including, for example, inorganic ionic salts made from calcium, potassium, sodium, or magnesium and inorganic acid salts made from hydrochloric acids, nitric acids, phosphoric acids, bromic acids, iodic acids, perchloric acids, or sulfuric acids; organic acid salts made from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, or vanillic acid; sulfonates made from methanesulfonic acid, ethanesulfonic acid, benzenesulfonic acid, p-toluenesulfonic acid, or naphthalenesulfonic acid; amino acid salts made from glycine, arginine, and lysine; or amine salts made of trimethylamine, triethylamine, ammonia, pyridine, or picoline, but the type of salts referred in the present disclosure is not limited by these salts listed.

The pharmaceutical composition of the present disclosure may be prepared in the form of a unit volume by preparation using a pharmaceutically acceptable carrier in accordance with a method that may be easily carried out by those skilled in the art to which the present disclosure pertains, or it may be prepared by introducing in a multi-capacity container.

The pharmaceutically acceptable carriers are those commonly used in preparation, including, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may additionally include lubricants, humectants, sweeteners, flavoring agents, emulsifiers, suspensions, and preservatives, in addition to the above ingredients.

As used herein, the content of the additives included in the pharmaceutical composition is not particularly limited and may be appropriately adjusted within the content range used in common preparation.

The pharmaceutical composition may be formulated in the form of one or more external agents selected from the group consisting of injectable formulations such as aqueous solutions, suspensions, and emulsions, pills, capsules, granules, tablets, creams, gels, patches, nebulizers, ointments, emplastrums, lotions, liniments, pastas, and cataplasmas.

The pharmaceutical composition of the present disclosure may include additional pharmaceutically acceptable carriers and diluents for formulation. The pharmaceutically acceptable carriers and diluents include, but are not limited to, excipients such as starch, sugars, and mannitol, fillers and extenders such as calcium phosphate, cellulose derivatives such as carboxymethylcellulose and hydroxypropyl cellulose, binders such as gelatin, alginate, and polyvinyl pyrrolidone, lubricants such as talc, calcium stearate, hydrogenated castor oil, and polyethylene glycol, disintegrating agents such as povidone and crospovidone, and surfactants such as polysorbate, cetyl alcohol, and glycerol. The pharmaceutically acceptable carriers and diluents may be biologically and physiologically friendly to a subject. Examples of diluents include, but are not limited to, brine, water-soluble buffers, solvents, and/or dispersion media.

The pharmaceutical composition of the present disclosure may be administered orally or parenterally (e.g., applied intravenously, subcutaneously, intraperitoneally, or topically) depending on the method of purpose. When administered orally, it may be formulated as tablets, troches, lozenges, water-soluble suspensions, oily suspensions, preparation powders, granules, emulsions, hard capsules, soft capsules, syrups, or elixirs. When administered parenterally, it may be formulated as an injection solution, suppositories, powders for respiratory inhalation, aerosols for sprays, ointments, powders for application, oils, and creams.

The dosage of the pharmaceutical composition of the present disclosure may vary depending on the patient's condition and weight, age, sex, health status, dietary constitution specificity, nature of the preparation, severity of disease, administration time of the composition, method of administration, duration or interval of administration, excretion rate, and drug form, and it may be appropriately selected by a person skilled in the art. For example, it may range from about 0.1 to 10,000 mg/kg but is not limited thereby, and administration may be performed once to several times a day.

The pharmaceutical composition may be administered orally or parenterally (e.g., applied intravenously, subcutaneously, intraperitoneally, or topically) depending on the desired method. The pharmaceutically effective amount and effective dose of the pharmaceutical composition of the present disclosure may vary depending on the preparation method of the pharmaceutical composition, the mode of administration, the administration time and/or administration route, and a person skilled in the art may easily determine and prescribe the effective dose for the desired treatment. The administration of the pharmaceutical composition of the present disclosure may be performed once a day or in several divided doses.

In addition, the present disclosure provides a preventive or health functional food composition for attention deficit/hyperactivity disorder (ADHD), including SNAP5114 and a foodologically acceptable food supplemental additive.

The present disclosure may be used generally as a commonly used food product.

The food supplemental additives include food additives that are common in the art, e.g., flavoring agents, savoring agents, colorants, fillers, and stabilizers, and are illustrated below.

The food composition of the present disclosure may be used as a health functional food. The term "health functional food" as used herein refers to food manufactured and processed with raw materials or ingredients having useful functionality for the human body in accordance with the Health Functional Food Act, and the term "functionality" as used herein refers to the intake to derive useful effectiveness in health care such as regulation of nutrients or physiological actions for the structure and function of the human body.

The food composition of the present disclosure may include common food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

The food composition of the present disclosure may be manufactured and processed in the form of tablets, capsules, powder, granules, liquids, and pills.

For example, hard capsule preparations among health functional foods in the form of capsules may be prepared by mixing and filling the composition according to the present disclosure in conventional hard capsules along with additives such as excipients, and the soft capsule preparations may be manufactured by mixing the composition according to the present disclosure with the additives such as excipients and then filling the same in capsule bases such as gelatin. The soft capsule preparations may include, if necessary, plasticizers such as glycerin or sorbitol, colorants, and preservatives.

The definition of terms for the excipient, binder, disintegrant, lubricant, flavor enhancer, and flavoring agent is described in documents known in the art and includes those having the same or similar functions. The type of food is not particularly limited and includes all health functional foods in the ordinary sense.

The term "prevention" as used herein refers to any action of suppressing or delaying diseases by administering the composition according to the present disclosure. The term "treatment" as used herein refers to any action that improves or favorably changes the symptoms of the disease by administering the composition according to the present disclosure. The term "amelioration" as used herein refers to any action that improves the bad state of the disease by making an individual intake the composition of the present disclosure or administering the same.

In addition, the present disclosure provides a biomarker composition for diagnosing attention deficit/hyperactivity disorder (ADHD), including GABA transporter-3 (GAT-3) or a gene encoding the same as an active ingredient.

In addition, the present disclosure provides a kit for diagnosing attention deficit/hyperactivity disorder (ADHD), including, as an active ingredient, a preparation capable of detecting an expression level of a GABA transporter-3 (GAT-3) protein or an expression level of a gene encoding the same.

The preparation capable of measuring the expression level of the protein is an antibody, peptide, aptamer, or compound that binds specifically to the protein, and the preparation capable of measuring the expression level of the gene is a primer or probe that binds specifically to the gene.

The antibodies include polyclonal antibodies, monoclonal antibodies, recombinant antibodies, and complete forms having two full-length light chains and two full-length heavy chains, as well as functional fragments of antibody molecules, e.g., Fab, F(ab'), F(ab')2, and Fv. The production of antibodies may be readily prepared using techniques widely known in the art to which the present disclosure pertains, and antibodies manufactured and commercially available may be used.

In addition, the level of protein may be measured by immunoassay or immunostaining. The method may be conducted in the form of a microchip or an automated microarray system capable of detecting the biomarker protein or fragments thereof in the sample.

The immunoassay or immunostaining method may include radioimmunoassay, radioimmunoprecipitation, immunoprecipitation, ELISA, capture-ELISA, inhibition or competition assay, sandwich assay, flow cytometry, immunofluorescence staining, and immunoaffinity purification.

Protein levels may be measured using multiple reaction monitoring (MRM), parallel reaction monitoring (PRM), sequential windowed data independent acquisition of the total high-resolution (SWATH), selected reaction monitoring (SRM), or immuno multiple reaction monitoring (iMRM). MRM is a method of determining the exact fragment of a material, breaking it in a mass spectrometer, and then selecting a specific ion once more from among ions that were once broken so as to obtain the number using a continuously connected detector.

The 'expression level of the gene' as used herein may be measured by using an antisense oligonucleotide, primer pair, or probe that binds specifically to the mRNA of the gene, and the preparation to measure the expression of the mRNA is selected from the group consisting of an antisense oligonucleotide, primer pair, probe, and a combination thereof that are specific to the gene. In other words, the detection of nucleic acids may be carried out by amplification reactions using one or more oligonucleotide primers that are hybridized to the nucleic acid molecule encoding the gene or a complementary object of the nucleic acid molecule, but is not limited thereto. For example, detection of mRNA using primers may be performed by amplifying the gene sequence using an amplification method such as PCR and then checking whether amplification occurs by methods known in the art, and may be measured by RT-PCR, competitive RT-PCR, quantitative RT-PCR, RNase protection assay, Northern blot, or DNA chip, but is not limited thereto.

The "probe" as used herein refers to nucleic acid fragments such as RNA or DNA corresponding to a few bases or hundreds of bases that may specifically form binding other than mRNA, and it is labeled so that the presence of a specific mRNA and the expression level may be identified. Probes may be prepared in the form of oligonucleotide probes, single strand DNA probes, double strand DNA probes, and RNA probes. The selection of the appropriate probe and the conditions for hybridization may be selected appropriately according to the technology known in the art.

The "primer" as used herein refers to a nucleic acid sequence having a short free 3' hydroxyl group, specifically, nucleic acid sequence capable of forming a base pair with a complementary template and acting as a starting point for replication of a template strand. The primer may initiate DNA synthesis in the presence of a reagent (i.e., DNA polymerase or reverse transcriptase) and four different nucleoside triphosphates for polymerization with involvement of an appropriate buffer and temperature. PCR conditions and a length of sense and antisense primers may be appropriately selected according to the techniques known in the art.

In addition, the present disclosure provides a method of providing information for diagnosis of attention deficit/hyperactivity disorder (ADHD), including measuring an expression level of a GABA transporter-3 (GAT-3) protein or an expression level of a gene encoding the same in a biological sample isolated from a subject suspected of having attention deficit/hyperactivity disorder (ADHD); and comparing the expression level with that of a biological sample isolated from a normal individual.

### Modes for Carrying Out the Invention

Hereinafter, to help understanding of the present disclosure, example embodiments will be described in detail. However, the following example embodiments are merely illustrative of the contents of the present disclosure, and the scope of the present disclosure is not limited to the following example embodiments. The example embodiments of the present disclosure are provided to more completely explain the present disclosure to those of ordinary skill in the art.

### Example 1. Preparation of animal model with ADHD

*Git1* gene-deficient hetero (+/-) mice were prepared as an animal model with attention deficit/hyperactivity disorder (ADHD). Wild-type mice and *GIT1* gene-deficient knock-out type (KO type) mice were crossed to obtain GIT1 gene-deficient hetero type. After making mating cages for among the obtained hetero type mice, genotyping was performed to isolate the hetero type mice.

SNAP5114 was administered intraperitoneally (I.P.) at a concentration of 50 ug/kg for 7 days prior to the experiment. SNAP5114 was dissolved in 10% DMSO and then used by dissolving in 90% saline just before the experiment.

Open field tests were performed to evaluate behavioral morphology and, in order to determine the characteristics of tonic GABA in the striatum, a representative brain region that controls hyperactivity, immunohistochemistry and electrophysiological measurements (patch clamp recording) were performed.

### Example 2. Evaluation on ADHD amelioration effect in animal model with ADHD

### 1) Fluorescent immunohistochemistry (fIHC)

To proceed with fluorescent immunohistochemistry (fIHC), perfusion was performed in mice. Primary perfusion was performed with phosphate-buffered saline (PBS), followed by fixation by adding 0.05% glutaraldehyde to 4% paraformaldehyde (PFA). Afterwards, the brain was collected from the skull of the mouse, which was then soaked in 4% PFA and stored at 4°C for half a day. The brains of the fixed mice were placed in a 30% sucrose solution and subjected to a drying process for two days. The dried brains were placed in the OCT compound and stored in a mold form at -80°C. After making the mold, the hippocampus and striatum were sectioned to a thickness of 30 µm using a cryotome. The sectioned slices were washed with PBS three times for 5 minutes and mixed with triton-X100 and normal goat serum for 1 hour. S 100b and GABA were used as primary antibodies and subjected to a reaction at 4°C for half a day. After the antibody reaction, washing was carried out with PBS three times for 5 minutes, followed by a reaction with fluorescence-conjugated secondary antibodies at room temperature for 2 hours. Washing was performed with PBS three times for 5 minutes, and brain tissues were stained on a slide glass using a DAKO mounting solution.

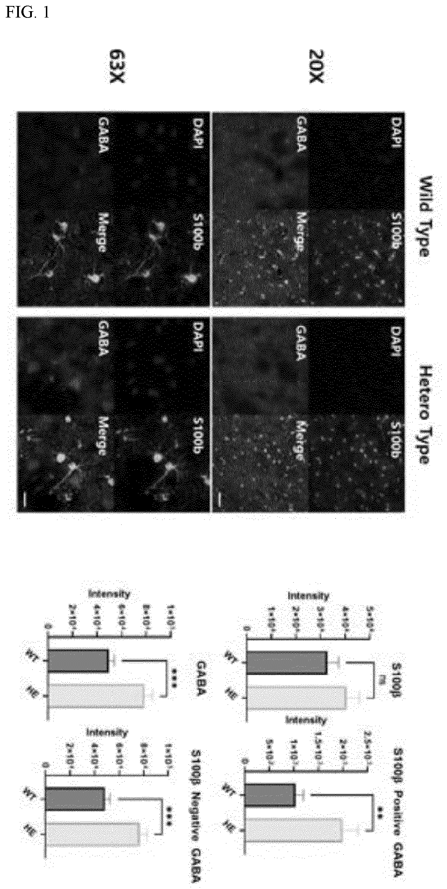

As shown in FIG. 1, an increase in an amount of GABA in astrocytes of Git1 hetero type (+/-) mice was observed. The image on the left side in FIG. 1 is a fluorescent image taken by confocal microscopy after fixing sections of brain striatal regions of Git1 wild type and hetero type mice and then performing fluorescent immunohistochemistry using specific antibodies, wherein DAPI represents the nucleus, s100b represents astrocytes, and GABA, gamma amino butyric acid, represents the main inhibitory neurotransmitter in the central nervous system. The graph on the right side in FIG. 1 is a confocal micrograph analyzed by Image J, and in the bar graph where the intensity of S100b was analyzed, no significance was derived, but the intensity of S100b positive GABA, i.e., GABA in the astrocytes, significantly increased in the Git1 hetero type. The overall GABA intensity and GABA levels outside astrocytes were also found to increase overall in the Git1 hetero type.

### 2) Electrophysiological measurement (patch clamp recording)

The electrophysiological measurement (patch clamp recording) was performed by sectioning the mouse striatum in a thickness of 300 um. After performing bubbling with a gas mixture of 95% O₂ and 5% CO₂, culture was followed in aCSF (unit in mM: 130 NaCl, 24 NaHCO₃, 1.25 NaH₂PO₄, 3.5 KCl, 1.5 CaCl₂, 1.5 MgCl₂, and 10 D(+)-glucose, pH 7.4). A measurement solution (including 135 CsCl, 4 NaCl, 0.5 CaCl₂, 10 HEPES, 5 EGTA, 2 Mg-ATP, 0.5 Na₂-GTP, and 10 QX-314. Adjusted to pH 7.2 using CsOH) was filled into a pipette to measure the electrophysiological state.

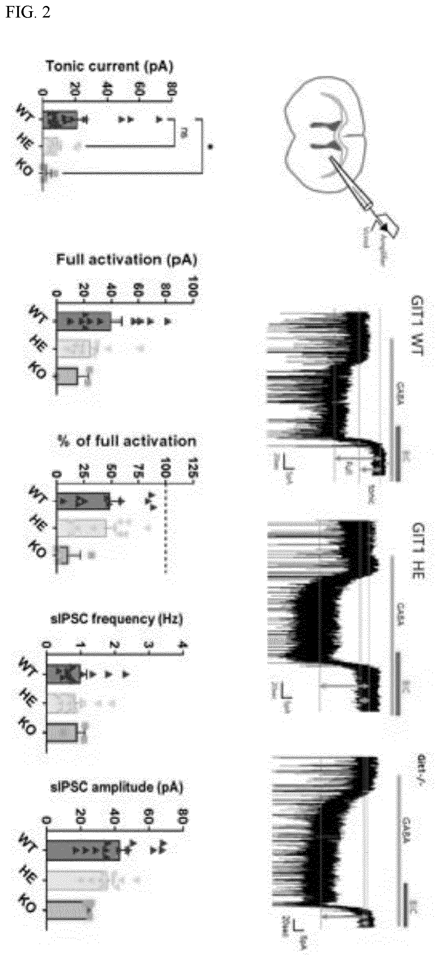

As shown in FIG. 2, the amount of tonic GABA measured in the striatum showed a tendency to decrease in the hetero type (+/-) and knock-out type (-/-) compared to the wild type (+/+). The upper left corner in FIG. 2 shows the location of the brain slice, including the striatum, a region of the brain where the patch clamp recording was performed, as well as a recording pipette. The graph on the upper right side in FIG. 2 shows the trace of tonic GABA currents measured in the medium spiny neuron of the striatum of the wild type, hetero type, and knock-out type. The gray bars indicate induction of a full activation current by treating GABA, while the red bars represent the time took for treatment of bicuculine, an antagonist of GABA-A receptors. The base line current was blocked by bicuculine treatment, and the full current and tonic current were measured as represented as a light blue arrow. The bottom in FIG. 2 shows graphs that the amplitude and frequency of all recorded traces were averaged to analyze significance, wherein a tonic GABA current amplitude (pA) on the bottom rightmost side showed values that are the largest in the wild type, significantly decreased in the hetero type, and the smallest in the knock-out type. In the case of a full activation current (pA) in the second graph and the % of full activation in the third graph showed a tendency similar to the tonic GABA current.

### Example 3. Evaluation on GAT-3 levels in animal model with ADHD

To proceed with fluorescent immunohistochemistry (fIHC), perfusion was performed in mice. Primary perfusion was performed with phosphate-buffered saline (PBS), followed by fixation by adding 0.05% glutaraldehyde to 4% paraformaldehyde (PFA). Afterwards, the brain was collected from the skull of the mouse, which was then soaked in 4% PFA and stored at 4°C for half a day. The brains of the fixed mice were placed in a 30% sucrose solution, and a drying process was performed for two days. The dried brains were placed in the OCT compound and stored in a mold form at -80°C. After making the mold, the hippocampus and striatum were sectioned in a thickness of 30 µm using a cryotome. The sectioned slices were washed with PBS three times for 5 minutes and mixed with triton-X100 and normal goat serum for 1 hour. S100b and GABA transporter-3 (GAT-3) were used as primary antibodies, followed by a redaction at 4°C for half a day. After the antibody reaction, washing was performed with PBS three times for 5 minutes, followed by a reaction with fluorescence-conjugated secondary antibodies at room temperature for 2 hours. Brain tissue was washed with PBS three times for 5 minutes, and brain tissue was stained on a slide glass using a DAKO mounting solution.

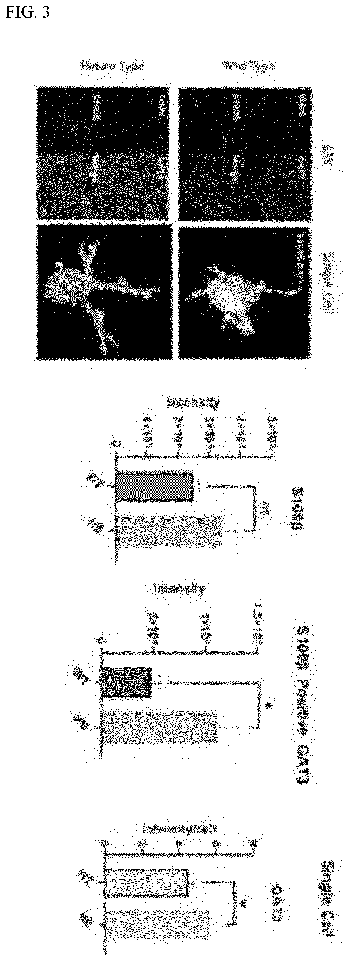

As shown in FIG. 3, as a result of identifying the amount of GABA transporter-3 (GAT-3) protein expressed in astrocytes, the amount of GAT-3 protein was found to be expressed at a higher level in Git1 hetero type (+/-) mice than in wild type (+/+) mice. The result above indicates that, in mice with attention deficit/hyperactivity disorder (ADHD), the amount of GABA in the astrocyte increases due to excessive expression of GABA transporter-3 (GAT-3), a transporter through which GABA enters astrocytes.

As shown on the left side in FIG. 3, the expression level of GAT-3 expressed in large quantities in astrocytes was found to increase significantly in the Git1 hetero type. As shown on the right side in FIG. 3, no significance was shown in the bar graph in which the intensity of S100b was analyzed, but the intensity of S100b positive GAT-3, i.e., the intensity of GAT3 in astrocytes, was found to significantly increase in the Git1 hetero type. GAT-3 expression was analyzed by photographing a single astrocyte at the single cell level using IMARIS, and as a result of analyzing by graph, it was found that GAT-3 expression in the Git1 hetero type increased significantly compared to the wild type.

### Example 4. Changes in hyperactivity upon treatment with GAT-3 inhibitors

Considering that changes in the amount of GABA associated with astrocytes in ADHD model mice are related to the transport protein that transports GABA into the astrocytes, behavioral experiments were conducted by treating the SNAP5114, an inhibitor of GABA transporter-3 (GAT-3). An open field test was performed to assess whether there is a behavioral improvement. Handling was performed 5 days before the open field test to minimize the stress that the mice feel from the experimenter. The mice were placed in the center of an open field test cage (30 X 30 X 30 cm), and its movement was measured for 10 minutes. The measured videos were analyzed for locomotor activity through ANYmaze.

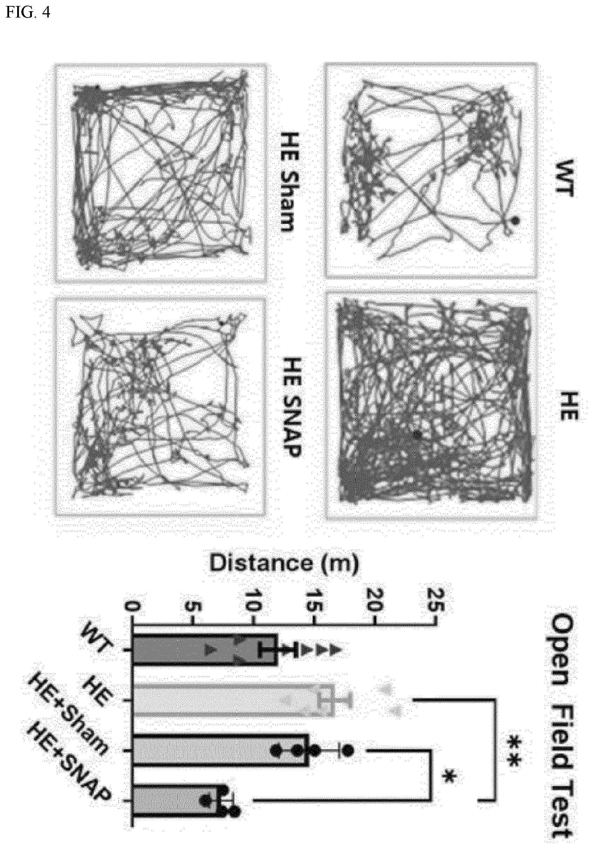

The left side in FIG. 4 shows tracking of the mouse movement in the open field, representing a distance and degree of activity of the mouse. The right side in FIG. 4 shows a graph of average values of the distance of movement for each individual employed in the experiment. As shown in FIG. 4, it was found that *Git1* hetero type (+/-) mice (HE) showed hyperactivity that has more movement compared to the wild type (+/+) mice (WT). When *Git1* hetero type (+/-) mice were treated with SNAP5114 (HE SNAP), hyperactivity was found to be ameliorated to the level of WT.

Having described in detail a specific part of the contents of the present disclosure above, it is clear for those skilled in the art that this specific description is merely a preferred example embodiment, and the scope of the present disclosure is not limited thereby. In other words, the substantial scope of the present disclosure is defined by the appended claims and their equivalents.

## Claims

1. A pharmaceutical composition for preventing or treating attention deficit/hyperactivity disorder (ADHD), comprising SNAP5114 or a pharmaceutically acceptable salt thereof as an active ingredient.

2. The pharmaceutical composition of claim 1, wherein the SNAP5114 is a compound represented by the following Chemical Formula 1:

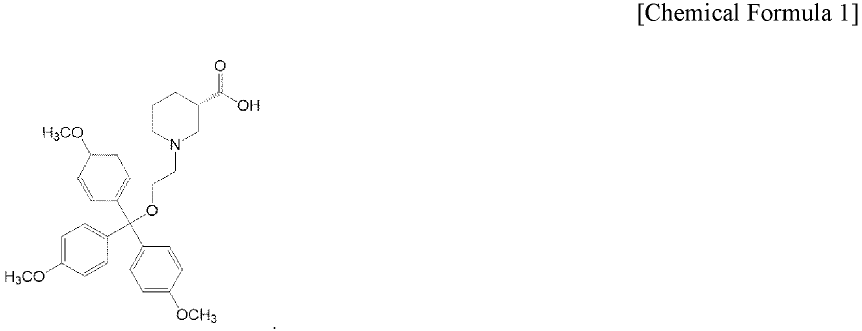

3. The pharmaceutical composition of claim 1, wherein the SNAP5114 ameliorates hyperactivity.

4. The pharmaceutical composition of claim 1, wherein the pharmaceutical composition comprises, for formulation, any one or more carriers selected from the group consisting of lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

5. A preventive or health functional food composition for attention deficit/hyperactivity disorder (ADHD), comprising SNAP5114 and a foodologically acceptable food supplemental additive.

6. The health functional food composition of claim 5, wherein the SNAP5114 is a compound represented by the following Chemical Formula 1:

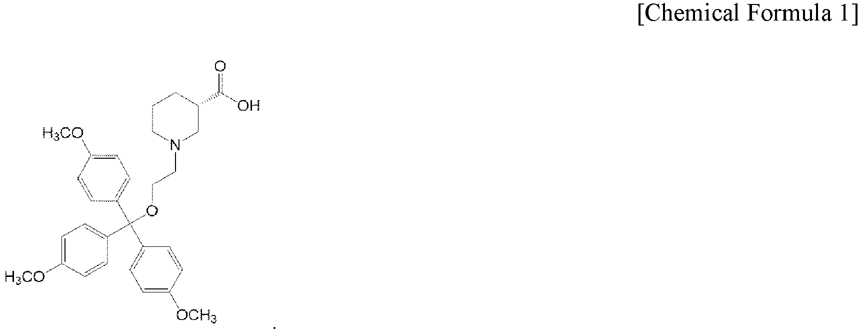

7. A biomarker composition for diagnosing attention deficit/hyperactivity disorder (ADHD), comprising GABA transporter-3 (GAT-3) or a gene encoding the same as an active ingredient.

8. A kit for diagnosing attention deficit/hyperactivity disorder (ADHD), comprising, as an active ingredient, a preparation capable of detecting an expression level of a GABA transporter-3 (GAT-3) protein or an expression level of a gene encoding the same.

9. The kit of claim 2, wherein the preparation capable of measuring the expression level of the protein is an antibody, peptide, aptamer, or compound that binds specifically to the protein.

10. The kit of claim 2, wherein the preparation capable of measuring the expression level of the gene is a primer or probe that binds specifically to the gene.

11. A method of providing information for diagnosis of attention deficit/hyperactivity disorder (ADHD), comprising:
measuring an expression level of a GABA transporter-3 (GAT-3) protein or an expression level of a gene encoding the same in a biological sample isolated from a subject suspected of having ADHD; and
comparing the expression level with that of a biological sample isolated from a normal individual.
